# EUROPEAN PATENT APPLICATION

(11) **EP 2 082 729 A1**
(43) Date of publication of application: **29.07.2009**
(21) Application number: 07828673.9
(22) Date of filing: 28.09.2007
(51) Int. Cl.: A61K 8/86, A61Q 19/00, A61Q 19/10

(54) **EXTERNAL PREPARATION FOR SKIN AND CLEANSING AGENT FOR SKIN**

(30) Priority: 29.09.2006 JP 2006269121; 29.09.2006 JP 2006269123
(71) Applicant: SHISEIDO COMPANY, LTD., Tokyo 104-8010 (JP); NOF Corporation, 20-3 Ebisu 4-chome, Shibuya-ku Tokyo 150-6019 (JP)
(72) Inventor: OHMORI, Takashi, Yokohama-shi, Kanagawa 224-8558 (JP); ISHIKUBO, Akira, Yokohama-shi, Kanagawa 224-8558 (JP); TESHIGAWARA, Takashi, Yokohama-shi, Kanagawa 224-8558 (JP); SUGIYAMA, Yuki, Yokohama-shi, Kanagawa 224-8558 (JP); YAGI, Eiichiro, Yokohama-shi, Kanagawa 224-8558 (JP); MARUYAMA, Kei-ichi, Kawasaki-shi, Kanagawa 210-0865 (JP); TAGUCHI, Sue, Kawasaki-shi, Kanagawa 210-0865 (JP); TEZUKA, Yoji, Kawasaki-shi, Kanagawa 210-0865 (JP)
(74) Representative: Lippert, Stachow & Partner
(86) International application number: PCT/JP2007/068931
(87) International publication number: WO 2008/041623

(57) **Abstract**

The present invention was made in view of the above-described circumstances, and an object of the invention is to provide an external skin preparation having a rough skin improving effect, excellent in texture in use, especially excellent in smoothness, free of sticky feeling, and excellent in stability. Another object is to provide a skin cleanser excellent especially in usability and a cosmetic removing effect.

That is, the external skin preparation of the present invention is **characterized by** comprising a block-type alkylene oxide derivative represented by the below-described formula (I).

[formula 1] Y-[O(EO)ₐ-(AO)_{b}-(EO)ₒ-R]ₖ (I)

(In the formula, Y is the residue given by removing hydroxyl groups from a polyhydric alcohol having 3 to 6 hydroxyl groups, and k is the number of hydroxyl groups of the polyhydric alcohol. EO is an oxyethylene group, AO is an oxyalkylene group having 3 to 6 carbon atoms, and they are attached in a block-type, respectively. The products, a×k, b×k, and c×k, are the average addition mole numbers of the oxyethylene group, the oxyalkylene group having 3 to 6 carbon atoms, and the oxyethylene group, respectively, and 0 ≦a×k≦ 100, 1≦b×k≦ 100, and 0≦c×k≦ 100, however, (a+c)×k>1. The percentage of all oxyethylene groups in formula (I) with respect to the sum of all oxyethylene groups and oxyalkylene groups having 3 to 6 carbon atoms in formula (I) is 10 to 80 mass %. Rs may be either identical to or different from each other, and they are hydrocarbon groups with 1 to 4 carbon atoms.)

The skin cleanser of present invention is **characterized by** comprising 0.01 to 70 mass % of a block-type alkylene oxide derivative represented by the above-described formula (I) and 0.1 to 20 mass % of a moisturizer.

## Description

### RELATED APPLICATIONS

This application claims the priority of Japanese Patent ApplicationNo. 2006-269121 filed on September 29, 2006, and Japanese Patent ApplicationNo. 2006-269123 filed on September 29, 2006, which are incorporated herein by reference.

### TECHNICAL FIELD

The present invention relates to an external skin preparation and a skin cleanser, and in particular, relates to the improvement of an external skin preparation and a skin cleanser containinga block-type alkylene oxide derivative as an active ingredient.

### BACKGROUND ART

The important factors for the evaluation of an external skin preparation include the function to maintain beautiful skin and the texture in use. On the other hand, typical means for improving the stability of base include the blending of a surfactant.

In order to maintain beautiful skin, it is important to prevent or improve skin roughness, keep the sulcus cutis and crista cutis orderly, and smooth the turnover of epidermal cells. The skin roughness is a skin trouble triggered by external factors such as dryness, UV light, and irritantsrepresented by surfactants or by internal factors such as hormonal imbalance. The roughening of the stratum corneum, due to a decrease in the stratum corneum barrier function, which is triggered by the above troubles, a decrease in the stratum corneum water content, acceleration of epidermal turnover, and formation of a squamous layer (scaling), is a big cosmetic issue.

As the active ingredient that displays prevention and improvement effects against skin roughness, moisturizers such as polyol compounds (e.g. sorbitol, propylene glycol, and glycerin) and mucopolysaccharides (e.g. hyaluronic acid) (for example, refer to patent literature 1), agents such as amino acids and tranexamic acid as the NMF (Natural Moisturizing Factor) (for example, refer to patent literature 2), and various extracts (for example, refer to patent literature 3) were traditionally blended in external skin preparations. In addition, the method to supplement the stratum corneumbarrier function with occlusion agents such as petrolatum ointment (for example, refer to patent literature 4) and the method to activate skin cells with vitamins, hormones, and the like have been used (for example, refer to patent literature 5).

However, when glycerin or other moisturizers were used, it was necessary to increase the blending quantity to increase the moisturizing effect and rough skin improving effect. As a result, there were cases in that the stability of base and the usability decreased.In addition, when such a base was applied to the skin, there were to-be-solved problems such as repulsion by sebum and poor skin compatibility. A polysaccharide precipitated in the formulations containing a large amount of alcohol. In the case of amino acids such as DL-threonine, there were drawbacks such as color development and malodor. The agent such as tranexamic acid has a problem in long-term stability. When occlusion agents such as petrolatum were used, there was a drawback in that an unpleasant texture such as an oily and sticky feeling was generated. Furthermore, when extracts, vitamins, hormones, and the like were used, there were to-be-solved problems such as safety issues related to side effects and long-term stability.

When an external skin preparation, and in particular, an emulsion is prepared, a surfactant is normally blended in order to improve the stability of base. As to the blending effect of surfactant, the blending of a substantial amount of surfactant could improve the stability of base; however, there was a case in that it contributed to the deterioration of the texture in use. If the blending quantity of surfactant is decreased,the stability of base tends to deteriorate. Thus, the balancing of the stability and the texture in use is one of the issues in the preparation of an emulsion.

In order to decrease the above-described texture in use, namely, a sticky feeling, a polyhydric alcohol or the like was blended. However, the texture could not be satisfactorily improved. Thus, the development of an ingredient that has a rough skin improving effect and excellent texture in use and that can improve the stability of base has been awaited.

As a makeup removing cleanser for skin external use, various types such as a lotion type, emulsion type, and oil type have been widely used.
Generally, in the makeup removing cleanser of lotion type, a mild nonionic surfactant or an amphoteric surfactant, which is not irritating to the skin, is blended as a cleansing surfactant. On the other hand, the cleansing effect of the oil type is achieved by using a solvent with solvent action as the main ingredient, and the cleansing effect of the emulsion type is achieved by dissolving and dispersing cosmetics mainly with the solvent action of oil and water.
Lately, so-called long-lasting cosmetics, which have good adherence to the skin and are resistant to makeup deterioration by water and sebum, have been developed. Therefore, in makeup removing cleansers also, measures such as increasing alcohol and surfactant contents or blending a surfactant with high cleansing action and a solvent with strong dissolving power have been taken in order to improve the cosmetic removing effect.

However, with the cleanser in which a surfactant with high cleansing action and a solvent with strong dissolving power are blended, there have been problems in that tingling and redness are causedafter cleansing or the irritation caused by later-used cosmetics is enhanced becauseof its high dissolving power. Thus, in order to avoid these problems and to realize a high cleansing power, a makeup removing cleanser containing a cyclic dimethylpolysiloxanehas been developed; however, there has been a to-be-solved problem such as difficulty in rising.
On the other hand, as an oil base that is highly safe and excellent in texture in use and skin compatibility, a specific polyethyleneglycol dialkyl ether is reported (for example, refer to patent literature 6). However, even when this polyethylene glycol dialkyl ether was blended, the makeup cleansing and rising were not wholly satisfactory.
In addition, a skin cleanser containing a specific alkylene oxide derivative, which is excellent in safety and usability and easy-to-rinse, has been reported (for example, refer to patent literature 7). However, even when this specific alkylene oxide derivative was blended, there was a to-be-solved problem such as a post-cleansing skin frictional feeling, which is considered to be due to the co-blended surfactant.

Patent literature 1: Japanese Unexamined Patent Publication No. H6-32728
Patent literature 2: Japanese Unexamined Patent Publication No. 2006-160758
Patent literature 3: Japanese Patent No. 3667291
Patent literature 4: Japanese Unexamined Patent Publication No. H9-1000225
Patent literature 5: Japanese Unexamined Patent Publication No. H7-277943
Patent literature 6: Japanese Unexamined Patent Publication No. H10-259112
Patent literature 7: Japanese Unexamined Patent Publication No. 2003-221310

### DISCLOSURE OF THE INVENTION

### PROBLEM TO BE SOLVED BY THE INVENTION

The present invention was made in view of the above-described circumstances, and an object of the invention is to provide an external skin preparation having a rough skin improving effect, excellent in texture in use, especially excellent in smoothness, free of sticky feeling, and excellent in stability. Another object is to provide a skin cleanser excellent especially in usability and a cosmetic removing effect.

### MEANS TO SOLVE THE PROBLEM

The present inventors have diligently studied to solve the above-described problems. As a result, the present inventors have found that an external skin preparation having a rough skin improving effect, excellent in texture in use, and excellent in stability can be obtained by blending a block-type alkylene oxide derivative with a specific structure in the external skin preparation.
In addition, the present inventors have found that when the above-described block-type alkylene oxide derivative is blended in a skin cleanser, the cleanser mixes well with cosmetics, the usability is excellent, for example, the rinsing during cleansing is easy and there is no sticky feeling or frictional feeling after cleansing, the cosmetic removing effect is excellent, and the skin irritation is low; thus leading to completion of the present invention.

That is, the external skin preparation of the present invention is characterized by comprising a block-type alkylene oxide derivative represented by the below-described formula (I).

[formula 1] Y-[O(EO)ₐ-(AO)_{b}-(EO)_{c}-R]ₖ (I)

(In the formula, Y is the residue given by removing hydroxyl groups from a polyhydric alcohol having 3 to 6 hydroxyl groups, and k is the number of hydroxyl groups of the polyhydric alcohol. EO is an oxyethylene group, AO is an oxyalkylene group having 3 to 6 carbon atoms, and they are attached in a block-type, respectively. The products, a×k, b×k, and c×k, are the average addition mole numbers of the oxyethylene group, the oxyalkylene group having 3 to 6 carbon atoms, and the oxyethylene group, respectively, and 0≦a×k≦100, 1≦b×k≦100, and 0≦c×k≦100, however, (a+c)×k>1. The percentage of all oxyethylene groups in formula (I) with respect to the sum of all oxyethylene groups and oxyalkylene groups having 3 to 6 carbon atoms in formula (I) is 10 to 80 mass %. Rs may be either identical to or different from each other, and they are hydrocarbon groups with 1 to 4 carbon atoms.)

In the above-described external skin preparation, it is preferable that AO of a block-type alkylene oxide derivative represented by the above-described formula (I) is an oxybutylene group.
In addition, in the above-described external skin preparation, it is preferable that the number a of the block-type alkylene oxide derivative represented by the above-described formula (I) is zero and that the addition order of AO and EO is (AO)-(EO) with respect to Y in the formula.

In the above-described external skin preparation, it is preferable to blend 0.01 to 70 mass % of a block-type alkylene oxide derivative represented by the above-described formula (I).
In addition, the present invention provides a skin roughness improving agent in which a block-type alkylene oxide derivative represented by the above-described formula (I) is an active ingredient.
In addition, the present invention provides a usability-improving agent in which a block-type alkylene oxide derivative represented by the above-described formula (I) is an active ingredient.
In the present invention, the above-described usability-improving agent means an agent that can improve texture in use, in particular, smoothness, and the absenceof sticky feeling when the agent is applied on the skin.

The skin cleanser of present invention is characterized by comprising 0.01 to 70 mass % of a block-type alkylene oxide derivative represented by the above-described formula (I) and 0.1 to 20 mass % of a moisturizer.
In the above-described skin cleanser, it is preferable that the content of a block-type alkylene oxide derivative with the above-described specific structure is 0.1 to 20 mass %.

In the above-described skin cleanser, it is preferable that the AO group of a block-type alkylene oxide derivative represented by the above-described formula (I) is an oxybutylene group.
In addition, in the above-described skin cleanser, it is preferable that the number a of the block-type alkylene oxide derivative represented by the above-described formula (I) is zero and that the addition order of AO and EO is (AO)-(EO) with respect to Y in the formula.
It is preferable that the above-described moisturizeris one or more selected from the group consisting of dipropylene glycol, propylene glycol, 1,3-butylene glycol, and glycerin.

### EFFECT OF THE INVENTION

According to the present invention, an external skin preparation having a rough skin improving effect, excellent in texture in use, especially excellent in smoothness, free of sticky feeling, and excellent in stability can be obtained by blending a block-type alkylene oxide derivative with a specific structure in the external skin preparation.
In addition, a skin cleanser having good mixability with cosmetics, excellent in usability such as easy rinsing during cleansing and no sticky feeling and frictional feeling after cleansing, excellent in the cosmetic removing effect, and low in skin irritation, can be obtained by blending 0.01 to 70 mass % of the above-described block-type alkylene oxide derivative and 0.1 to 20 mass % of a moisturizer.

### BEST MODE FOR CARRYING OUT THE INVENTION

In the following, a preferable embodimentof the present invention will be described.
The external skin preparation and the skin cleanser of the present invention comprise a block-type alkylene oxide derivative represented by the below-described formula (I).

[formula 2] Y-[O(EO)ₐ-(AO)_{b}-(EO)_{c}-R]ₖ (I)

In the alkylene oxide derivative represented by the above-described formula (I), Y is the residue given by removing hydroxyl groups from a polyhydric alcohol having 3 to 6 hydroxyl groups, and k is the number of hydroxyl groups of the polyhydric alcohol and k is 3 to 6. Examples of the compounds having 3 to 6 hydroxyl groups include glycerin, trimethylolpropane, and hexylene glycol (k = 3); erythritol and pentaerythritol (k = 4); xylitol (k = 5); and sorbitol and inositol (k = 6). The basic skeleton of the polyoxyalkylene glycol/polyethylene glycol copolymer alkyl ether derivative, which is blended in the skin external preparation of the present invention, is the residue given by removing hydroxyl groups from a mixture of one or more polyhydric alcohols having 3 to 6 hydroxyl groups. The basic skeleton of the block-type alkylene oxide derivative, which is blended in the external skin preparation and the skin cleanser of the present invention, is the residue given by removing hydroxyl groups from a mixture of one or more polyhydric alcohols having 3 to 6 hydroxyl groups.
In the present invention, it is preferable that Y is the residue given by removing hydroxyl groups from a polyhydric alcohol having 3 to 4 hydroxyl groups, namely, it is desirable to satisfy 3≦k≦4. If k is 2 or lower, the smooth feeling tends to be poor when blended in the external skin preparation. If k is 7 or higher, a sticky feeling tends to be generated. When blended in the skin cleanser, if k is 2 or lower, the smooth feeling of the skin tends to be poor after cleansing. If k is 7 or higher, a sticky feeling of the skin tends to be generated after cleansing.

EO is an oxyethylene group having 2 carbon atoms. AO is an oxyalkylene group having 3 to 6 carbon atoms, and the specific examples include an oxypropylene group, an oxybutylene group, an oxyisobutylene group, an oxy-t-butylene group, an oxypentylene group, and an oxyhexylene group. AO is preferably an oxypropylene group or an oxybutylene group, and more preferably an oxybutylene group.

The product b×k is the average addition mole number of AO, and it is 1≦b×k≦100, and preferably 3≦b×k≦70. The products a×k and c×k are the average addition mole numbers of EO, and they are 0≦a×k≦100 and 0≦c×k≦100, and preferably 3≦a×k≦70 and 3≦c×k≦70. In addition, the preferable range of the average addition mole number of all oxyethylene groups in the above-described formula (I) is 1<(a+c)×k≦200, and more preferably 6≦(a+c)×k≦140. AO is a hydrophobic domain in the block-type alkylene oxide derivative of the present invention. If the product b×k is zero, the stability tends to be poor when blended in an external skin preparation. If the product b×k exceeds 100, the moist texture in use tends to be poor. When blended in a skin cleanser, if the product b×k is zero or exceeds 100, the cosmetic removing effect is low, and the skin moisturizing effect feeling after cleansing tends to be poor.
In addition, if the product a×k or c×k is zero, the stability tends to be poor when blended in an external skin preparation. If the product a×k or c×k exceeds 100, a sticky feeling tends to be generated. When blended in a skin cleanser, if the product a×k or c×k is zero, the expected cosmetic removing effect cannot be realized. If the product a×k or c×k exceeds 100, a sticky feeling of the skin tends to be generated after cleansing.

The percentage of all EO in the above-described formula (I) with respect to the sum of AO and EO in the above-described formula (I) is preferably 10 to 80 mass %, and more preferably 20 to 70 mass %. When blended in an external skin preparation, if the above-described percentage is smaller than 10 mass %, the moist texture in use tends to be poor. If the above-described percentage exceeds 80 mass %, a sticky feeling tends to be generated. When blended in the skin cleanser, if the above-described percentage is smaller than 10 mass %, the cosmetic removing effect tends to be poor. If the above-described percentage is larger than 80 mass %, the cosmetic removing effect is low, and a sticky feeling of the skin tends to be generated after cleansing.
The addition mode of AO and EO is block-type, and the addition order may be any of the orders (AO)-(EO), (EO)-(AO), and (EO)-(AO)-(EO)with respect to Y in the formula. In the present invention, the order (AO)-(EO) with respect to Y in the formula is especially preferable. In the case that the order is (AO)-(EO) with respect to Y in the formula, the number a in the formula is zero.

R is a hydrocarbon group having 1 to 4 carbon atoms, and the examples of hydrocarbon groups include a methyl group, an ethyl group, an n-propyl group, an isopropyl group, an n-butyl group, a sec-butyl group, and a tert-butyl group. In the present invention, a methyl group or an ethyl group is preferable. If the number of carbonatoms is larger than 5, the moist texture in use tends to be poor when blended in an external skin preparation. When blended in a skin cleanser, the skin moisturizing effect feeling after cleansing tends to be poor. In the block-type alkylene oxide derivative that is blended in the external skin preparation or the skin cleanser of the present invention, Rs in one molecule may be either identical to or different from each other, and either one kind of block-type alkylene oxide derivative having identical Rs in one molecule or a mixture of two kinds or more of block-type alkylene oxide derivatives having different Rs may be used.

Specific examples of alkylene oxide derivatives that can be blended into the skin cleanser of the present invention include, POB(30)POE(30)glyceryl trimethyl ether, POB(30)POE(35)glyceryl trimethyl ether, POB(17)POE(28)glyceryl trimethyl ether, POB(27)POE(45)glyceryl trimethyl ether, POB(14)POE(34)glyceryl trimethyl ether, POB(22)POE(55)glyceryl trimethyl ether, POB(19)POE(55)glyceryl trimethyl ether, POB(40)POE(80)glyceryl trimethyl ether, POB(80)POE(40)glyceryl trimethyl ether, POB(30)POE(30)glyceryl triethyl ether, POB(30)POE(35)glyceryl trimethyl ether, POB(14)POE(34)glyceryl triethyl ether, POB(30)POE(30)glyceryl tripropyl ether, POE(30)POP(30)glyceryl trimethyl ether, POE(35)POP(40)glyceryl trimethyl ether, and POE(41)POP(48)glyceryl trimethyl ether.
The above-described POE, POP, and POB are the abbreviations of polyoxyethylene, polyoxypropylene, and polyoxybutylene, respectively. Hereinafter, they may be abbreviated as such.

An alkylene oxide derivative that is blended in the external skin preparation or the skin cleanser of the present invention can be produced by a publicly known method. For example, the alkylene oxide derivative can be obtained by the addition polymerization of ethylene oxide and an alkylene oxide having 3 to 6 carbon atoms to a compound such as a polyhydric alcohol having hydroxyl groups and subsequent etherification with an alkyl halide in the presence of an alkaline catalyst.

By blending the thus prepared alkylene oxide derivative with a specific structure into an external skin preparation, the external skin preparation, having a rough skin improving effect, excellent in texture in use, especially excellent in smoothness, free of sticky feeling, and excellent in stability, can be obtained. In the external skin preparation of the present invention, the blending quantity of the above-described alkylene oxide derivative with a specific structure is preferably 0.01 to 70 mass % with respect to the total composition, and more preferably 0.1 to 20 mass %. If the blending quantity is less than 0.01 mass %, the manifestation of the blending effect may not be satisfactory. If the blending quantity exceeds 70 mass %, a sticky feeling may be generated.

In addition, by blending the above-described alkylene oxide derivative with a specific structure and a moisturizer, a skin cleanser, excellent in usability such as easy rinsing during cleansing and no sticky feeling and frictional feeling after cleansing, excellent in mixability with cosmetics, excellent in the cosmetic removing effect, and low in skin irritation, can be obtained. In the skin cleanser of the present invention, the blending quantity of the above-described block-type alkylene oxide derivative is normally 0.01 to 70 mass % with respect to the total skin cleanser, preferably 0.1 to 20 mass %, and especially preferably 1 to 10 mass %. If the blending quantity is less than 0.01 mass %, the manifestation of the blending effect may not be satisfactory. If the blending quantity exceeds 70 mass %, a sticky feeling may be generated after cleansing.

Specific examples of moisturizers that can be blended into the skin cleanser of the present invention include polyethyleneglycol, dipropylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile, salts, dl-pyrrolidone carboxylates, short-chain soluble collagen, diglycerin (EO)PO adduct, chestnut rose extract, yarrow extract, and melilot extracts, N-lauroyl-N'-carboxymethyl-N'-(2-hydroxyethyl)ethylenediamine sodium, N-myristoyl-N'-carboxymethyl-N'-(2-hydroxyethyl)ethylenediamine sodium, N-palmitoyl-N'-carboxymethyl-N'-(2-hydroxyethyl)ethylenediamine sodium, N-lauroyl-N'-carboxymethyl-N'-(2-hydroxyethyl)ethylenediamine potassium, and N-lauroyl-N'-carboxymethyl-N'-(2-hydroxyethyl)ethylenediamine magnesium. In particular, one or more selected from the group consisting of dipropylene glycol, propylene glycol, 1,3-butylene glycol, and glycerin are preferable.

In the external skin preparation and the skin cleanser of the present invention, the components normally used in cosmetics, pharmaceuticals, or quasi-drugs can be suitably blended, in addition to the above-described essential components, so far as the effect of the present invention is not undermined. Specific blendable components are listed below. That is, the external skin preparation and the skin cleanser of the present invention can be produced by suitably adding the below-described components in addition to the above-described essential components.

As powder components, for example, inorganic powder (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate, calcium phosphate, fluorine apatite, hydroxyapatite, ceramic powder, metallic soap (for example, zinc myristate, calcium palimitate, and aluminum stearate), and boron nitride, etc); organic powder (for example, polyamide resin powder (nylon powder), polyethylenepowder, polymethylmethacrylate powder, polystyrene powder, styrene-acrylic acid copolymer powder, benzoguanamine resin powder, poly(tetrafluroethylene) powder, and cellulose powder, etc); inorganic white family pigment (for example, zinc oxide, etc); inorganic red family pigment (for example, iron oxide (colcothar), and iron titanate, etc); inorganic brown family pigment (for example, γ-iron oxide, etc); inorganic yellow family pigment (for example, yellow iron oxide, and loess, etc); inorganic black family pigment (for example, black iron oxide, and lower titanium oxide, etc); inorganic purple family pigment (for example, mango violet, cobalt violet, etc); inorganic green family pigment (for example, chrome oxide, chrome hydroxide, cobalt titanate, etc); inorganic blue family pigment (for example, ultramarine, iron blue, etc); pearl pigment (for example, titanium oxide coated mica, titanium oxide coated bismuth oxychloride, titanium oxide coated talc, colored titanium oxide coated mica, bismuth oxychloride, argentine, etc); metal powder pigment (aluminum powder, copper powder, etc); organic pigment such as zirconium, barium, or aluminum lake (for example, organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (for example, chlorophyll, β-carotene, etc), etc.

As liquid fat, for example, avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, chinese wood oil, jojoba oil, germ oil, triglycerol, can be listed.

As solid fat, for example, cacao butter, coconut oil, horse fat, hydrogenated coconut oil, palm oil, beef fat, mutton suet, hydrogenated beef fat, palm kerneloil, lard, beef bones fat, Japan wax kernel oil, hardened oil, hoof oil, Japan wax, hydrogenated caster oil, can be listed.

As waxes include ,for example, beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, and POE hydrogenated lanolin alcohol ether, can be listed.

As hydrocarbon oils include liquid paraffin, ozocerite, squalene, pristane, paraffin, ceresin. squalane, vaseline, microcrystalline wax, can be listed.

As higher fatty asid include, for example, lauric acid, myristic acid, palmitic acid, stearic acid, behenic acid, oleic acid, undecylenic acid, tallic acid, isostearic acid, linoleic acid, linolenic acid, eicosapentaenoic acid(EPA),docosahexaenoic acid(DHA) and the like.

Higher alcohol include, for example, linear alcohol (for example, lauryl alcohol, cetyl alcohol, stearyl alcohol, behenyl alcohol, myristyl alcohol, oleyl alcohol, and cetostearyl alcohols; branched-chain alcohols (for example, monostearylglycerin ether (batyl alcohol),2-decyltetradecinol, lanolin alcohol, cholesterol, phytosterol, hexyldodecanol, isostearyl alcohol, and octyldodecanol) and the like.

As ester oils, isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoiso stearate, neopentyl glycol dicaprate, diisostearyl malate, glycerol di-2-heptyl undecanoate, trimethyrolpropane tri-2-ethyl hexanoate, trimethyrolpropane triisostearate, tetra-2-ethyl hexanoate pentaerythritol, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate can be listed.

Silicone oil include, for example, chain polysiloxane (for example, dimethylpolysiloxane, methylphenylpolysiloxane, diphenylpolysiloxane); cyclic polysiloxane (for example, octamethylcyclotetrasiloxane, decamethylcyclopentasiloxane, dodecamethylcyclohexasiloxane), silicone resins having a three dimensional network structure, silicone rubbers, various modified polysiloxanes (for example, amino-modified polysiloxane, polyether-modified polysiloxane, alkyl-modified polysiloxane, fluorine-modified polysiloxane) and the like.

In addition, various surfactants may be blended in the external skin prepara tion and the skin cleanser of the present invention.
Anionic surfactants include, for example, fatty acid soap (for example, sodium lau rate, sodium palmitate); higher alkyl sulfate ester salt (for example, sodium lauryl sulf ate, potassium lauryl sulfate); alkyl ether sulfate ester salt (for example, POE lauryl su lfate triethanolamine, sodium POE lauryl sulfate); N-acyl sarcosinic acid (for example, sodium lauroyl sarcocinate) ; higher fatty acid amide sulfonate (for example, sodium N-myristoyl-N-methyl taurine, sodium coconut oil fatty acid methyl tauride, sodium lau rylmethyl tauride); phosphate ester salt (sodium POE oleylether phosphate, POE stearyl ether phosphate); sulfosuccinate (for example, sodium di-2-ethylhexyl sulfosuccinate, so dium monolauroyl monoethanolamide polyethylene sulfosuccinate, sodium lauryl polypr opylene glycol sulfosuccinate); alkylbenzene sulfonate (for example, sodium linear dode cylbenzene sulfonate, triethanolamine linear dodeylbenzene sulfonate, linear dodecylbenz ene sulfonate); higher fatty acid ester sulfate ester salt (for example, sodium hydrogen ated gryceryl cocoate sulfate), N- acyl glutamate (for example, monosodium N-lauroyl glutamate, disodium N-stearoyl glutamate, monosodium N-myristoyl-L-glutamate); sulfon ated oil (for example, Turkey red oil); POE alkyl ether carboxylic acid; POE alkyl ar yl ether carboxylate; α-olefine sulfonate; higher fatty acid ester sulfonate, secondary al cohol sulfate ester salt, higher fatty acid alkylolamide sulfate ester salt, sodium lauroyl monoethanolamide succinate; N-palmitoyl asparaginate ditriethanolamine; sodium casei n and the like.

Cationic surfactants include, for example, alkyltrimethyl ammonium salt (for example, stearyltrimethyl ammonium chloride, lauryltrimethyl ammonium chloride); alk ylpyridinium salt (for example, cetylpyridinium chloride); distearyldimethyl ammonium chloride; dialkyldimethyl ammonium salt; poly (N,N'-dimethyl-3,5-methylenepiperidiniu m) chloride; alkyl quaternary ammonium salt; alkyldimethylbenzyl ammonium salt; alk ylisoquinolinium salt; dialkylmorphonium salt; POE alkylamine; alkylamine salt; polya mine fatty acid derivative; amyl alcohol fatty acid derivative; benzalkonium chloride; b enzethonium chloride and the like.

Ampholytic surfactants include, for example, imidazoline base ampholytic su rfactants (for example, sodium 2-undecyl-N,N,N-(hydroxyethylcarboxymethyl)-2-imidazol ine, 2-cocoyl-2-imidazolinium hydroxide-1-carboxyethyloxy)-2-sodium salt; betaine base surfactants (for example, 2-heptadecyl-N-carboxymethyl-N-hydroxyethylimidazolinium be taine, lauryldimethyl aminoacetate betaine, alkyl betaine, amidobetaine, sulfobetaine) an d the like.

Lipophilic nonionic surfactants include for example, sorbitan fatty acid ester s (for example, sorbitan monooleate, sorbitan monoisostearate, sorbitan monolaurate, so rbitan monopalmitate, sorbitan monostearate, sorbitan sesquioleate, sorbitan trioleate, dig lycerol sorbitan penta-2 ethylhexylate, diglycerol sorbitan tetra-2 ethylhexylate); glycery 1 polyglyceryl fatty acids (for example, glyceryl monocotton oil fatty acid, glyceryl mo noerucate, glyceryl sesquioleate, glyceryl monostearate, glyceryl α, α'-oleate pyroglutam ate, glyceryl monostearate malate); propylene glycol fatty acid esters (for example, pro pylene glycol monostearate); hydrogenated caster oil derivative; glyceryl alkyl ether an d the like.

Hydrophilic nonionic surfactants include, for example, POE sorbitan fatty acid esters (for example, POE sorbitan monooleate, POE sorbitan monostearate, POE sorbitan monooleate, POE sorbitan tetraoleate); POE sorbit fatty acid esters (for example, POE sorbit monolaurate, POE sorbit monooleate, POE sorbit pentaoleate, POE sorbit monostearate), POE glyceryl fatty acid esters (for example, POE monooleatesuch as POE glyceryl monostearate, POE glyceryl monoisostearate, POE glyceryl triisostearate);POE fatty acid esters (for example, POE distearate, POE monodioleate, ethyleneglycol distearate); POE alkyl ethers (for example, POE lauryl ether, POE oleyl ether, POE stearyl ether, POE behenyl ether, POE-2-octyldodecyl ether, POE cholestanol ether); puluronic types (for example, Puluronic), POE/POP alkyl ethers (for example, POE/POP cetyl ether, POE/POP 2-decyltetradecyl ether, POE/POP monobutyl ether, POE/POP hydrogenated lanoline,POE/POP glycerin ether); tetra POE/tetra POP ethylenediamine condensation products (for example, Tetronic); POE castor oil hydrogenated castor oil derivatives (for example, POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivatives (for example, POE sorbitol beeswax); alkanolamides (for example, coconut oil fatty acid diethanolamide, lauric acid monoethanolamide, fatty acid isopropanolamide); POE propyleneglycol fatty acid esters; POE alkyl amines; POE fatty acid amides; sucrose fatty acid esters; alkylethoxydimethylamine oxide; trioleyl phosphoric acid and the like.

As natural water-soluble polymer include, for example, plant-based polymer (for example, gum Arabic,gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid), microorganisms based polymer (for example, xanthan gum, dextran, succinoglycan, pullulan, etc), animal-based polymer (for example, collagen, casein, albumin, gelatine, etc) and the like.

As semisynthetic water-soluble polymer include, for example, starch-based polymer (for example, carboxymethyl starch, methylhydroxypropyl starch, etc), cellulosic polymer (methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, micrclrystalline cellulose, cellulose powder, etc), algin acid base polymer, (for example, alginate sodium, propylene glycol ester alginate, etc), and the like.

As synthetic water-soluble polymer include, for example, vinyl base polymer (for example, polyvinyl alcohol, polyvinyl methyl ether, polyvinylpyrrolidone, carboxyvinylpolymer, etc); polyoxyethylene base polymer (for example, polyethylene glycol 20,000, 40,000 and 60,000); acrylic polymer (fro example, sodium polyacrylate, polyethylacrylate, polyacrylamide, etc); polyethyleneimine;cationpolymer, and the like.

As plasticizer include, for example, gum Arabic,carrageenan, gum karaya, gum tragacanth, guar gum, gum Arabic,locust bean gum, quince seed (cydonia oblonga), casein, dextrine, gelatine, sodium pectate, alginate sodium, methylcellulose, CMC, hydroxyethylcellulose, hydroxypropylcellulose, PVA, PVM, PVP, sodium polyacrylate, carboxyvinylpolymer, locust bean gum, guar gum, tamarind gum, dialkyldimethylammonium cellulose sulfate, xanthan gum, aluminium magnesium silicate, bentonite,hectorite, aluminium magnesium silicate (veegum), laponite, silicic anhydride, and the like.

Examples of ultraviolet light absorbers include benzoic acid family ultraviolet light absorbers (for example, p-aminobenzoic acid (hereinafter abbreviated as PABA),PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid family ultraviolet light absorbers (for example, homomenthyl N-acetylanthranilate etc.); salicylic acid family ultraviolet light absorbers (for example, amyl salicylate, menthyl salicylate, homomenthyl salicylate, octyl salicylate, phenyl salicylate, benzyl salicylate, p-isopropanolphenyl salicylate, etc.); cinnamic acid family ultraviolet light absorbers (for example, octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc.); benzophenone family ultraviolet light absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.); 3-(4'-methylbenzylidene)-d,1-camphor and 3-benzylidene-d,1-camphor; 2-phenyl-5-methylbenzoxazol; 2,2'-hydroxy-5-methylphenylbenzotriazol,2-(2'-hydroxy-5'-t-octylphenyl) benzotriazol, and 2-(2'-hydroxy-5'-methylphenylbenzotriazol; dibenzalazine; dianisoylmethane; 4-methoxy-4'-t-butyldibenzoylmethane; and 5-(3,3-dimethyl-2-norbornylidene)-3-pentane-2-one.

As lower alcohol include, for example, ethanol, propanol, isopropanol,isobutyl alcohol, t-butyl alcohol, and the like.

As the orgaminc amine include, for example, monoethanolamine, diethanolamine, morpholine, triisopropanolamine, 2-amino-2-methyl-1,3-propanediol, 2-amino-2-methyl-1-propanol, and the like.

As chelate agents include, for example, 1-hydroxyethane-1, 1-diphosphonic acid, 1-hydroxyethane, 1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

As anti-oxidants include, tocopherols, dibutyl hydroxy toluene, butyl hydroxy anisole, and gallic acid esters, and the like. As anti-oxidant aids include, for example, phosphoric acid, citric acid, ascorbic acid, maleic acid, malonic acid, succinic acid, fumaric acid, cephalin, hexamethaphosphate, phytic acid, and ethylene diamine tetra-acetic acid, and the like.

In particular, as any components to the external skin preparation include, for example, below-described moisturizers, polyhydric alcohol, monosaccharides, and oligosaccharides, and the like..
Examples of moisturizers include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salts, dl-pyrrolidone carboxylates, short-chain soluble collagen, diglycerin (EO)PO adduct, chestnut rose extract, yarrow extract, and melilot extract.

As polyhydric alcohol include, for example, dihydric alcohol (for example, ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (for example, glycerin, trimethylolpropane, etc); tetrahydric alcohol (for example, such as pentaerythritol such as 1,2,6-hexanetriol); pentahydric alcohol (for example, xylitol, etc); hexahydric alcohol (for example, sorbitol, mannitol, etc); polyhydric alcohol polymer (for example, diethyleneglycol, triethylene glycol, polypropylene glycol, tetraethylene glycol, diglycerin, polyethyleneglycol, triglycerin, tetraglycerin, polyglycerin, etc); dihydric alcohol alkyl ethers (for example, ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (for example, diethyleneglycol monomethyl ether, diethylene glycol monoethyl ether, diethyleneglycol monombutyl ether, diethyleneglycol dimethyl ether, diethyleneglycol diethyl ether, diethyleneglycol butyl ether, diethyleneglycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (for example, ethylene glycol monomethyl ether acetate, ethylene glycol monobutyl ether acetate, ethylene glycol monophenyl ether acetate, ethylene glycol diadipate, ethylene glycol disaccinate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (for example, chimil alcohol, selachyl alcohol, batyl alcohol, etc); sugar alcohol (for example, sorbitol, maltitol, maltotriose, mannitol, sucrose, erythritol,glucose, fructose,starch sugar, maltose, xylitose, starch sugar hydrogenated alcohol, etc); glysolid, tetrahydrofurfurylalcohol; POE-tetrahydrofurfuryl alcohol; POP-POE-butyl ether; tripolyoxypropylene glycerin ether; POP- glycerin ether; POP- glycerin ether phosphoric acid; POP-POE-pentaerythritol ether; polyglycerin, and the like.

As monosaccharides include, for example, triose (for example, D-glyceryl aldehyde, dihydroxyacetone, etc); tetrose (for example, D-erythrose, D- erythrulose, D-threose, erythritol,etc); pentaose (for example, L-arabinose, D-xylose, L-lyxose, D-arabinose, D-ribose, D-ribulose, D-xylulose, L-xylulose, etc); hexalose (for example, D-glucose, D-talose, D-psicose, D-galactose, D-fructose, L-galactose, L-mannose, D-tagatose, etc); heptose (for example, aldoheptose, heplose); octose (for example, octulose); deoxy sugar (for example, 2-deoxy-D-ribose, 6-deoxy-L-galactose, 6-deoxy-L-mannose);amino sugar (for example, D-glucosamine, D-galactosamine, sialic acid, amino uronic acid, muramic acid, etc); uronic acid (for example, D-grucuronic acid, D-mannuronic acid, L-guluronic acid, garacturonic acid, L-iduronic acid, etc) and the like.

As oligosaccharides include, for example, sucrose, guntianose, umbelliferose, lactose, planteose, isolignose type, α,α-trehalose, raffinose, lignose type, umbellicine, stachyose, verbascose type, and the like. As polysaccharide include, for example, cellulose, quince seed, chondroitinsulfate, starch, galactan, dermatan sulfate, glycogen, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.
As amino acids include, for example, neutral amino acid (for example threonine, cysteine, etc); basic amino acid (for example, hydroxylysine, etc) and the like. As amino acid derivatives include, for example, sodium acyl sarcosine (sodium lauroyl sarcosine), acyl glutamate, sodium acyl β-alanine, glutathione, pyrrolidone carboxylate, and the like.

In particular, as any components to the external skin creanser include, for example, below-described amino acids, polymer emulsion, pH modifiers, and vitamine group, and the like.
As amino acids include, for example, neutral amino acid (for example threonine, cysteine, etc); basic amino acid (for example, hydroxylysine, etc) and the like. As amino acid derivatives include, for example, sodium acyl sarcosine (sodium lauroyl sarcosine), acyl glutamate, sodium acyl β-alanine, glutathione, pyrrolidone carboxylate, and the like.

As polymer emulsion include, for example, acrylate resin emulsion, ethyl polyacrylate emulsion, liquid acryl resin, polyacrylalkylester emulsion, polyvinyl acrylate resin emulsion, natural rubber latex, and the like.

As pH modifiers include ,buffers such as lactic acid-sodium lactic acid, citric acid-sodium citric acid, succinic acid- sodium succinic acid and the like. As vitamine group include, for example, vitamineA, B1, B2, B6, C, E, and their derivatives, pantothenicacid, and their derivatives, biotin and the like.

As other containable compositions include, for example, antiseptic agent (ethylparaben, butylparaben, etc); lightening agent (for example, placental extract, saxifrage extract, arbutin, etc); antiphlogistics (for example, glycyrrhizinic acid derivatives, salicylic acid derivatives, hinokitiol, zinc oxide, allantoin, etc); blood circulation promotion agent (for example, nonyl acid vanillyl amide, nicotine acid, nicotine acid benzyl , nicotine acid benzyl ester, tocopherol nicotinate, nicotine acid β-butoxy ester, nicotine acid β-butoxyethyl ester, minoxidil, or their analogs, vitamine E type, γ-oryzanol, alkoxycarbonylpyridine N-oxide, capronium chloride, acetylcholine and their derivatives, capsaicin, zingerone, cantharides tincture, ichthammol, tannic acid, α-borneol, tocopherol nicotinate, meso-inositol hexanicotinate, cyclandelate, cinnarizine, tolazoline, acetylcholine, verapamil, cepharanthine, etc); various extract (for example, ginger, cork tree bark, oat, Japanese coptis, lithospermum, peony, swertia herb, birch, sage, loquat, carrot, aloe, mallow, iris, grape, mugwort, sponge gourd, lily, saffron, cnidium rhizome, ginger , hypericum, restharrow, garlic, red pepper, citrus unshiu, Japanese angelica, Japanese tree peoney, seaweed, etc); activator agent (for example, pantothenyl ethyl ether, nicotinamide, biotin, pantothenicacid, royal jelly, photosenstizer, cholesterol derivatives) antiseborrheric agent, (for example, pyridoxine, sulfur, thianthl, etc); anti-inflammatory agent (for example, tranexamic acid, thiotaurine, hypotaurine, etc) and the like.

The external skin preparation and skin cleanser of the present invention can be in any form, and the examples include a solution form, solubilized form, emulsion form, dispersed powder form, water-oil double-layer form, and water-oil-powder triple-layer form.
The external skin preparation of the present invention can be any form such as lotion, gel, mist, spray, mousse, roll-on, or stick.
The skin cleanser of the present invention is suitably used for the cleansing of the skin, in particular, for the cleansing of the skin after makeup or after the application of sunscreen. It is preferable to take a gel from or cream form.

Hereinafter, the present invention will be more concretely described by examples. However, the present invention is not limited by these examples. The blending quantity is expressed in mass % unless otherwise noted.

### [Embodiment 1]

Initially, the evaluation tests were conducted for the usability and stability of the external skin preparation of the present invention.
At first, the evaluation methods used for the present tests of the external skin preparation will be described. The application sites of the external skin preparation are cheeks.
The composition of the conventional external skin preparation used as the control is as follows.

| (Water phase components) | |
|---|---|
| (1) Glycerin | 10.0 % mass |
| (2) Carboxyvinylpolymer | 0.1 |
| (3) Potassium hydroxide | 5.0 |
| (4) Sorbitol | 3.0 |
| (5) Ethanol | 4.0 |
| (6) Purified water | reminder |
| (Oil phase components) | |
| (7) Liquid paraffin | 5.0 |
| (8) Glyceryltri-2-ethylhexanoate | 4.0 |
| (9) Cetyl isooctanoate | 2.0 |
| (10) Antiseptic | proper quantity |
| (11) Perfume | proper quantity |

### Evaluation (1): Smoothness of the skin

Concerning the smoothness of the skin during use and after use of the external skin preparation, we asked 10 professional panelists to actually use test examples of the external skin preparation shown below and the conventional external skin preparation as the control (composition containingglycerin as the moisturizer) and to determine the score based on the below-described rating criteria. Here, the score was determined by setting the score of the control external skin preparation to be zero. The average value was calculated by dividing the sum of scores of each panelist by the number of panelists, and the evaluation results were obtained based on the below-described evaluation criteria.

### Rating criteria

+3: A very smooth feeling is present compared with the control external skin preparation.
+2: A smooth feeling is present compared with the control external skin preparation.
+1: Some smooth feeling is present compared with the control external skin preparation.
0: Neither applies.
-1: A smooth feeling is hardly present compared with the control external skin preparation.
-2: No smooth feeling is present compared with the control external skin preparation.
-3: Absolutely no smooth feeling is present compared with the control external skin preparation.

### Evaluation criteria

A: The average value of 10 panelists is +1.5 points or higher.
B: The average value of 10 panelists is 0 point or higher and less than 1.5 points.
C: The average value of 10 panelists is -1.5 points or higher and less than 0 point.
D: The average value of 10 panelists is less than -1.5 points

### Evaluation (2): Absence of a sticky feeling

Concerning the absenceof a sticky feeling after the application of the external skin preparation on the skin, we asked 10 professional panelists to actually use test examples of the external skin preparation shown below and the conventional external skin preparation as the control (composition containingglycerin as the moisturizer) and to determine the score based on the below-described rating criteria. Here, the score was determined by setting the score of the control external skin preparation to be zero. The average value was calculated by dividing the sum of scores of each panelist by the number of panelists, and the evaluation results were obtained based on the below-described evaluation criteria.

### Rating criteria

+3: Absolutely no sticky feeling is present compared with the control external skin preparation.
+2: No sticky feeling is present compared with the control external skin preparation.
+1: Almost no sticky feeling is present compared with the control external skin preparation,.
0: Neither applies.
-1: Some sticky feeling is present compared with the control external skin preparation.
-2: A sticky feeling is present compared with the control external skin preparation.
-3: A very sticky feeling is present compared with the control external skin preparation.

### Evaluation criteria

A: The average value of 10 panelists was +1.5 points or higher.
B: The average value of 10 panelists was 0 point or higher and less than 1.5 points.
C: The average value of 10 panelists was less than -1.5 points.

### Evaluation (3): Moist feeling (Moisturizing effect feeling)

Concerning the moist feeling (moisturizing effect feeling) after 2 hours of the application of the external skin preparation, we askhe average value of 10 panelists was -1.5 points or higher and less than 0 point.
D: Ted 10 professional panelists to actually use test examples of the external skin preparation shown below and the conventional external skin preparation as the control (composition containingglycerin as the moisturizer) and to determine the score based on the below-described rating criteria. Here, the score was determined by setting the score of the control external skin preparation to be zero. The average value was calculated by dividing the sum of scores of each panelist by the number of panelists, and the evaluation results were obtained based on the below-described evaluation criteria.

### Rating criteria

+3: A very moist feeling is present compared with the control external skin preparation.
+2: Moist feeling is present compared with the control external skin preparation.
+1: Some moist feeling is present compared with the control external skin preparation.
0: Neither applies.
-1: A moist feeling is hardly present compared with the control external skin preparation.
-2: No moist feeling is present compared with the control external skin preparation.
-3: Absolutely no moist feeling is present compared with the control external skin preparation.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### Evaluation (4): Test for rough skin improving effect

The test for a rough skin improving effect was conducted, according to the following method, by 10 subjects having rough skin on the face (region: cheeks)by applying external skin preparations.
Test method: Different external skin preparations (external skin preparation of each test example and the control external skin preparation) were applied on the right and left cheeks once a day on consecutive days for a week, and the score was determined, on the next day after the end of the test period, based on the below-described rating criteria. Here, the score was determined by setting the score of the control external skin preparation to be zero. The average value was calculated by dividing the sum of scores of each panelist by the number of panelists, and the evaluation results were obtained based on the below-described evaluation criteria.

### Rating criteria

+3: There is a feeling that the rough skin is improved very much compared with the control external skin preparation.
+2: There is a feeling that the rough skin is improved compared with the control external skin preparation.
+1: There is a feeling that the rough skin is slightly improved compared with the control external skin preparation.
-1: There is hardly a feeling that the rough skin is improved compared with the control external skin preparation.
-2: There is no feeling that the rough skin is improved compared with the control external skin preparation.
-3: There is absolutely no feeling that the rough skin is improved compared with the control external skin preparation.
0: Neither applies.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### Evaluation (5): Skin irritation test

A 24-hour occlusive patch test was performed on the medial side of the upper arm of 10 subjects, and then the average value was calculated based on the following rating criteria. The evaluation criteria were as described below.

### Rating criteria

0 point: No abnormality was observed.
1 point: Slight redness was observed.
2 points: Redness was observed.
3 points: Redness and papules were observed.

### Evaluation criteria

A: The average value of 10 panelists was less than 0.15 points.
B: The average value of 10 panelists was 0.15 points or higher and less than 0.2 points.
C: The average value of 10 panelists was 0.2 points or higher and less than 0.3 points.
D: The average value of 10 panelists was 0.3 points or higher.

### Evaluation (6): Stability

For each test example of the external skin preparation, the stability evaluation was conducted, by the visual observation immediately after the production and by the visual observation after being filled in a glass bottle and allowed to stand for 6 weeks at 50 °C, based on the following criteria.
A: No change was observed in appearance.
B: A slight separation of the oil phase or the water phase was observed.
C: A substantial separation of the oil phase or the water phase was observed.

The present inventors producedexternal skin preparations, by the ordinary method, with the blending compositions shown in Tables 1 to 3, and the evaluation tests were conducted in the above-described evaluation items (1) to (6).
Initially, external skin preparations in which the conventional moisturizers and a block-type alkylene oxide derivative were blended, respectively, were investigated. The results are shown in Table 1.

The block-type alkylene oxide derivative in the below-described table has a structure of the below-described formula (II). In the case that a+c+e=30 and b+d+f=30, for example, it will be denoted by (BO)₃₀(EO)₃₀.

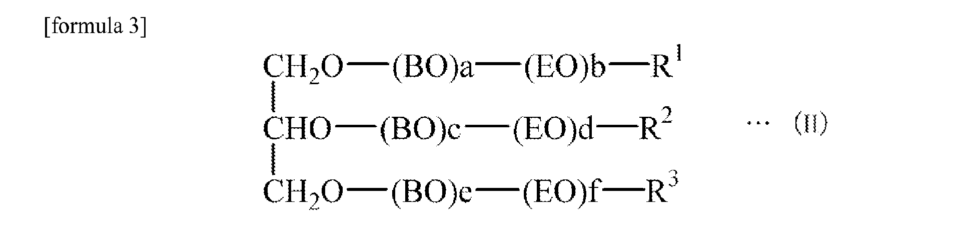

**[Table1]**

| | control | Test Example | | | |
|---|---|---|---|---|---|
| | | 1 | 2 | 3 | 4 |
| (Water phase components) | | | | | |
| (BO)₃₀(EO)₃O, R^{1∼3}=CH₃, block polymer | - | 5.0 | - | - | - |
| Glycerin | 10.0 | - | 5.0 | - | - |
| 1,3-butylene glycol | - | - | - | 5.0 | - |
| Carboxyvinylpolymer | 0.1 | 0.1 | 0.1 | 0.1 | 0.1 |
| Potassium hydroxide | 5.0 | 5.0 | 5.0 | 5.0 | 5.0 |
| Sorbitol | 3.0 | 3.0 | 3.0 | 3.0 | 3.0 |
| Ethanol | 4.0 | 4.0 | 4.0 | 4.0 | 4.0 |
| Purified water | Reminder | Reminder | Reminder | Reminder | Reminder |
| (Oil Phase components) | | | | | |
| Liquid Paraffin | 5.0 | 5.0 | 5.0 | 5.0 | 5.0 |
| Glyceryl tri-2-ethylhexanoate | 4.0 | 4.0 | 4.0 | 4.0 | 4.0 |
| Cetyl isooctanoate | 2.0 | 2.0 | 2.0 | 2.0 | 2.0 |
| Antiseptic | proper quantity | proper quantity | proper quantity | proper quantity | proper quantity |
| Perfume | proper quantity | proper quantity | proper quantity | proper quantity | proper quantity |
| Evaluation (1): Smoothness ofthe skin | - | A | C | B | C |
| Evaluation (2): Absence of a sticky feeling | - | A | B | B | A |
| Evaluation (3): Moist feeling | - | A | C | C | D |
| Evaluation (4): Test for rough skin improving effect | - | A | C | C | C |
| Evaluation (5): Skin irritation test | B | A | A | B | B |
| Evaluation (6): Stability | C | A | B | B | C |

As seen from the results in Table 1, the control external skin preparation in which glycerin was blended as the moisturizer had poor stability. When the blending quantity of glycerin was suppressed (Test Example 2), the moisturizing effect represented by a moist feeling and the rough skin improving effect decreased.
On the other hand, the external skin preparation in which a block-type alkylene oxide derivative was blended (Test Example 1) was excellent in all of the above-described evaluations, and it was well-balanced in the texture in use, moisturizing effect, rough skin improving effect, and the stability. In the composition in which the moisturizers such as glycerin and an alkylene oxide derivative were not blended (Test Example 4), the moisturizing effect and rough skin improving effect could not naturally be expected, and the stability was poor.

Subsequently, the present inventors investigated various alkylene oxide derivatives. The results are shown in Table 2.

**[Table2]**

| | Test Example | | | | | | | | | | |
|---|---|---|---|---|---|---|---|---|---|---|---|
| | 5 | 6 | 7 | 8 | 9 | 10 | 11 | 12 | 13 | 14 | 15 |
| (Water phase components) | | | | | | | | | | | |
| (BO)₁₄(EO)₃₄, R^{1∼3}=CH₃, block polymer | 5.0 | - | - | - | - | - | - | - | - | - | - |
| (BO)₂₂(EO)₅₅, R^{1∼3}=CH₃, block polymer | - | 5.0 | - | - | - | - | - | - | - | - | - |
| (BO)₃₀(EO)₃₀, R^{1∼3}=CH₃, block polymer | - | - | 5.0 | - | - | - | - | - | - | - | - |
| (BO)₄₀(EO)₈₀, R^{1∼3}=CH₃, block polymer | - | - | - | 5.0 | - | - | - | - | - | - | - |
| (BO)₈₀(EO)₄₀, R^{1∼3}=CH₃, block polymer | - | - | - | - | 5.0 | - | - | - | - | - | - |
| (BO)₃₀(EO)₃₀, R^{1∼3}=C₄H₉, block polymer | - | - | - | - | - | 5.0 | - | - | - | - | - |
| (EO)₅₇, R^{1∼3}=CH₃ | - | - | - | - | - | - | 5.0 | - | - | - | - |
| (BO)₃₄, R^{1∼3}=CH₃ | - | - | - | - | - | - | - | 5.0 | - | - | - |
| (BO)₃₀(EO)₃₀, R^{1∼3}=H, block polymer | - | - | - | - | - | - | - | - | 5.0 | - | - |
| (BO)₃₀(EO)₃₀, R^{1∼3}=H, random polymer | - | - | - | - | - | - | - | - | - | 5.0 | - |
| POE (60) hydrogenated castor oil | - | - | - | - | - | - | - | - | - | - | 5.0 |
| Carboxyvinylpolymer | 0.1 | 0.1 | 0.1 | 0.1 | 0.1 | 0.1 | 0.1 | 0.1 | 0.1 | 0.1 | 0.1 |
| Potassium hydroxide | 5.0 | 5.0 | 5.0 | 5.0 | 5.0 | 5.0 | 5.0 | 5.0 | 5.0 | 5.0 | 5.0 |
| Sorbitol | 3.0 | 3.0 | 3.0 | 3.0 | 3.0 | 3.0 | 3.0 | 3.0 | 3.0 | 3.0 | 3.0 |
| Ethanol | 4.0 | 4.0 | 4.0 | 4.0 | 4.0 | 4.0 | 4.0 | 4.0 | 4.0 | 4.0 | 4.0 |
| Purified water | Reminder | Reminder | Reminder | Reminder | Reminder | Reminder | Reminder | Reminder | Remainder | Reminder | Reminder |

| (Oil Phase components) | | | | | | | | | | | |
|---|---|---|---|---|---|---|---|---|---|---|---|
| Liquid Paraffin | 5.0 | 5.0 | 5.0 | 5.0 | 5.0 | 5.0 | 5.0 | 5.0 | 5.0 | 5.0 | 5.0 |
| Glyceryl tri-2-ethylhexanoate | 4.0 | 4.0 | 4.0 | 4.0 | 4.0 | 4.0 | 4.0 | 4.0 | 4.0 | 4.0 | 4.0 |
| Cetyl isooctanoate | 2.0 | 2.0 | 2.0 | 2.0 | 2.0 | 2.0 | 2.0 | 2.0 | 2.0 | 2.0 | 2.0 |
| Antiseptic | proper quantity | proper quantity | proper quantity | proper quantity | proper quantity | proper quantity | proper quantity | proper quantity | proper quantity | proper quantity | proper quantity |
| Perfume | proper quantity | proper quantity | proper quantity | proper quantity | proper quantity | proper quantity | proper quantity | proper quantity | proper quantity | proper quantity | proper quantity |
| Evaluation (1): Smoothness of the skin | A | A | A | B | A | A | D | B | D | A | B |
| Evaluation (2): Absence of a sticky feeling | A | A | A | B | A | A | C | B | D | A | D |
| Evaluation (3): Moist feeling | A | A | A | A | B | B | B | D | B | A | C |
| Evaluation (4): Test for rough skin improving effect | A | A | A | A | B | B | C | C | C | A | D |
| Evaluation (5): Skin irritation test | A | A | A | A | B | B | B | C | C | A | B |
| Evaluation (6): Stability | B | B | B | B | B | B | C | C | B | C | B |

When the blended alkylene oxide derivative consists of only the oxyethylene part or only the oxybutylene part (Test Examples 11 and 12), the stability was very poor because these materials do not function as a surfactant. In addition, in Test Example 11 in which an alkylene oxide derivative with only the oxyethylene part was blended, the texture in use and the rough skin improving effect were especially poor. In Test Example 12 in which an alkylene oxide derivative with only the oxybutylene part is blended, the moisturizing effect feeling and the rough skin improving effect were poor.

In Test Example 13 in which an alkylene oxide derivative with terminal hydrogen atoms is blended, the texture in use and the rough skin improving effect were not satisfactory. In addition, there was a problem of skin irritation. When a random-type alkylene oxide derivative was blended (Test Example 14), the stability was poor because the function as a surfactant is low.
When POE (60) hydrogenated castor oil, which has been traditionally used as a surfactant, was blended, for the purpose of stabilization, in the external skin preparation (Test Example 15), the moisturizing effect feeling and the rough skin improving effect could not be achieved.
On the other hand, the test examples in which various block-type alkylene oxide derivatives were blended (Test Examples 5 to 10) were excellent in all evaluations (1) to (6).

As is clear from the above-described results, when a block-type alkylene oxide derivative with a specific structure is blended in an external skin preparation, a base having a rough skin improving effect, excellent in texture in use, especially excellent in smoothness, free of sticky feeling, excellent in moisturizing effect feeling, and excellent in stability becauseof an additionalsurfactant function, can be obtained.
Next, the results for the investigation of the preferable blending quantity, in an external skin preparation, of a block-type alkylene oxide derivative with a specific structure are shown in Table 3.

**[Table3]**

| | Test Example | | | | |
|---|---|---|---|---|---|
| | 16 | 17 | 18 | 19 | 20 |
| (BO)₁₄(EO)₃₄, R^{1∼3}=CH₃, block polymer | 0.01 | 0.1 | 20.0 | 30.0 | 70.0 |
| Ethanol | 4.0 | 4.0 | 4.0 | 4.0 | 4.0 |
| Nicotinic acid | 0.2 | 0.2 | 0.2 | 0.2 | 0.2 |
| Sodium Pyrrolidonecarboxylate | 0.5 | 0.5 | 0.5 | 0.5 | 0.5 |
| Antiseptic | proper quantity | proper quantity | proper quantity | proper quantity | proper quantity |
| Perfume | proper quantity | proper quantity | proper quantity | proper quantity | proper quantity |
| Purified water | Reminder | Reminder | Reminder | Reminder | Reminder |
| Evaluation (1): Smoothness ofthe skin | B | A | A | B | B |
| Evaluation (2): Absence of a sticky feeling | B | A | A | B | B |
| Evaluation (3): Moist feeling | B | A | A | A | A |
| Evaluation (4): Test for rough skin improving effect | B | A | A | A | A |
| Evaluation (5): Skin irritation test | B | A | A | A | A |

As is clear from the results in Table 3, when the blending quantity of a block-type alkylene oxide derivative with a specific structure is in the range of 0.01 to 70 mass %, the rough skin improving effect and the moisturizing effect feeling are excellent compared with the conventional external skin preparation. When the texture in use such as the smoothness of the skin and the absenceof sticky feeling is considered, it is especially preferable that the blending quantity of the above-described alkylene oxide derivative is 0.1 to 20 mass %.

In the following, some synthesis examples of alkylene oxide derivatives, which were blended in the external skin preparations of the above-described test examples, are shown.

### <Synthesis Example 1>

### Synthesis of polyoxybutylene(30 mol)polyoxyethylene(30 mol)trimethylglyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.
The hydroxyl value of the compound that was obtained by purifying a sample taken before the methyl chloride reaction was 49, and the hydroxyl value of alkylene oxide derivative 1 was 0.4. The ratio of the number of terminal hydrogen atoms and the number of the terminal methyl groups was 0.008; thus the hydrogen atoms are almost completely replaced with methyl groups.

### <Synthesis Example 2>

### Synthesis of polyoxyethylene(57 mol)trimethylglyceryl ether (alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2508 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water.In addition, filtration was conducted to remove the salt formed after the treatment, and the alkylene oxide derivative was obtained.
The hydroxyl value of the compound that was obtained by purifying a sample taken before the methyl chloride reaction was 66, and the hydroxyl value of alkylene oxide derivative 1 was. The ratio of the number of terminal hydrogen atoms and the number of the terminal methyl groups was 0.60.009; thus the hydrogen atoms are almost completely replaced with methyl groups.

### <Synthesis Example 3>

### Synthesis of polyoxybutylene(30 mol)polyoxyethylene(30 mol)glyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.

### <Synthesis Example 4>

### Synthesis of polyoxybutylene(30 mol)polyoxyethylene(30 mol)tributylglyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, 800 g of potassium hydroxide was loaded, the inside of the system was replaced with dry nitrogen, 1200 g of butyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.
The hydroxyl value of the compound that was obtained by purifying a sample taken before the methyl chloride reaction was 50, and the hydroxyl value of alkylene oxide derivative 1 was 1.5. The ratio of the number of terminal hydrogen atoms and the number of the terminal butyl groups was 0.03; thus the hydrogen atoms are almost completely replaced with butyl groups.

### <Synthesis Example 5>

### Synthesis of polyoxybutylene(30 mol)polyoxyethylene(30 mol)glyceryl ether (random-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, a mixture of 2160 g of butylene oxide and 1320 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the random-type alkylene oxide derivative was obtained.
The hydroxyl value of the compound that was obtained by purifying a sample taken before the methyl chloride reaction was 47, and the hydroxyl value of the obtained compound, random-type alkylene oxide derivative 5, was 0.5. The ratio of the number of terminal hydrogen atoms and the number of the terminal methyl groups was 0.011; thus the hydrogen atoms are almost completely replaced with methyl groups.

In the following, the formulation examples of the external skin preparation of the present invention were listed. However, the technical scope of the present invention is not limited by these. The obtained external skin preparations had a rough skin improving effect, and they were excellent in texture in use, especially excellent in smoothness, free of sticky feeling, and excellent in emulsion stability.

### Example 1 : Milky lotion

| (Components) | (% by weight) |
|---|---|
| Phase A | |
| (1) Squalane | 4.0 |
| (2) Oleylolate | 2.5 |
| **(**3) Petrolatum | 1.5 |
| (4) POB(30)POE(35) trimethyl glycerylether | 2.0 |
| 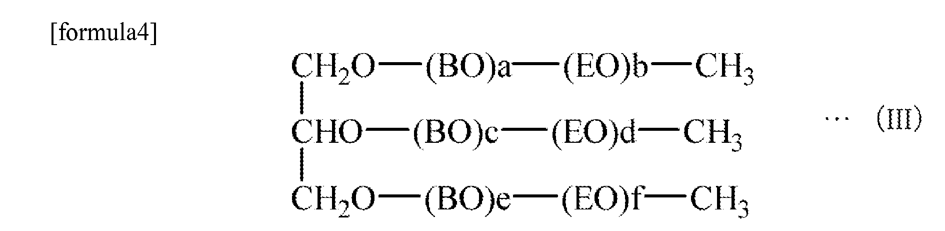 | |
| (In the formula (III), a+c+e=30, b+d+f=35, BO is an oxybuthylene group, EO is an oxyethylene group.) | |
| (5) Evening primrose oil | 0.2 |
| (6) Perfume | 0.1 |
| (7) Antiseptic | proper quantity |

| Phase B | |
|---|---|
| (8) 1,3-butylene glycol | 1.5 |
| (9) Ethanol | 2.0 |
| (10) Carboxyvinylpolymer | 0.2 |
| (11) Potassium hydroxide | 0.1 |
| (12) L-Arginine L-aspartate | 0.01 |
| (13) Edetic acid | 0.05 |
| (14) Purified water | reminder |

### (Process)

Phase A and phase B were dissolved, respectively, by heating to 70 °C, phase A was added to phase B, and the emulsification was carried out with an emulsifying machine. The desired milky lotion was obtained by cooling the obtained emulsion with a heat exchanger.

### Example 2 : Cream

| (Components) | (% by weight) |
|---|---|
| Phase A | |
| (1) Stearic acid | 10.0 |
| (2) Stearyl alcohol | 3.5 |
| (3) Butyl stearate | 6.0 |
| (4) POB(30)POE(35) trimethyl glycerylether | 1.5 |
| (In the above-formula (III), a+c+e =30, b+d+f=35, BO is an oxybuthylene group, EO is an oxyethylene group.) | |
| (5) Glycerylmonoisostearate | 2.5 |
| (6) Vitamin E acetate | 0.5 |
| (7) Vitamin A palmitate | 0.1 |
| (8) Macadamia oil | 0.5 |
| (9) Perfume | 0.15 |
| (10) Antiseptic | proper quantity |

| Phase B | |
|---|---|
| (11) Glycerin | 6.0 |
| (12) 1,2-Pentanediol | 2.0 |
| (13) Sodium hyaluronate | 1.5 |
| (14) Potassium hydroxide | 2.0 |
| (15) Magnesium ascorbyl phosphate | 1.5 |
| (16) L-Arginine L-aspartate | 0.01 |
| (17) Trisodium edetate | 0.05 |
| (18) Purified water | reminder |

### (Process)

Phase A and phase B were dissolved, respectively, by heating to 70 °C, phase A was added to phase B, and the emulsification was carried out with an emulsifying machine. The desired cream was obtained by cooling the obtained emulsion with a heat exchanger.

### Example 3 : Skin lotion

| (Components) | (% by weight) |
|---|---|
| Phase A | |
| (1) Ethanol | 5.0 |
| (2) POB(32)POE(52) trimethyl glycerylether | 0.2 |
| (In the above-formula (III), a+c+e =32, b+d+f=52, BO is an oxybuthylene group, EO is an oxyethylene group.) | |
| (3) 2-Ethylhexyl-P-dimethylaminobenzoate | 0.1 |
| (4) Antiseptic | proper quantity |
| (5) Perfume | 0.1 |

| Phase B | |
|---|---|
| (6) Dodium DL-pyrrolidonecarboxylate | 0.3 |
| (7) Nicotinamide | 0.2 |
| (8) Dimorpholino pyridazinone | 0.1 |
| (9) Aloe extract | 0.2 |
| (10) Purified water | reminder |

### (Process)

Phase A and phase B were dissolved, respectively, phase A was added and solubilized to phase B, and the desired skin lotion was obtained.

### Example 4 : Foundation

| (Components) | (% by weight) |
|---|---|
| Phase A | |
| (1) Cetanol | 3.5 |
| (2) Deodorized lanolin | 4.0 |
| (3) Jojoba oli | 5.0 |
| (4) Petrolatum | 2.0 |
| (5) Squalane | 6.0 |
| (6) Glycerylmonoisostearate | 2.5 |
| (7) POB(17)POE(28) trimethyl glycerylether | 1.5 |
| (In the above-formula (III), a+c+e =17, b+d+f=28, BO is an oxybuthylene group, EO is an oxyethylene group.) | |
| (8) Pyridoxine tripalmitate | 0.1 |
| (9) Perfume | 0.3 |
| (10) Antiseptic | proper quantity |

| Phase B | |
|---|---|
| (11) Propylene glycol | 10.0 |
| (12) Spherical nylon powder | 2.0 |
| (13) Silicone treated titanium dioxide | 5.0 |
| (14) Silicone treated iron oxide | 2.0 |
| (15) Silicone treated mica | 1.0 |
| (16) Metal soap treated talc | 2.0 |
| (17) Trisodium edetate | 0.5 |
| (18) Purified water | reminder |

### (Process)

Phase A and phase B were dissolved, respectively, by heating to 70 °C, phase A was added to phase B, and the emulsification was carried out with an emulsifying machine. The desired foundation was obtained by cooling the obtained emulsion with a heat exchanger.

### Example 5 : Lotion mask

| (Components) | (% by weight) |
|---|---|
| Phase A | |
| (1) Ethanol | 8.0 |
| (2) POB(41)POE(48) trimethyl glycerylether | 0.5 |
| (In the above-formula (III), a+c+e = 41, b+d+f=48, BO is an oxybuthylene group, EO is an | |
| oxyethylene group.) | |
| (3) Menthyl lactate | 0.002 |
| (4) Perfume | 0.01 |
| (5) Antiseptic | proper quantity |

| Phase B | |
|---|---|
| (6) Birch extract | 0.2 |
| (7) Caustic potash | proper quantity |
| (8) Purified water | reminder |

### (Process)

A lotion was prepared by adding phase A to phase B, and the desired lotion mask was obtained by further impregnating the lotion into a nonwoven cloth.

Subsequently, the skin cleanser of the present invention was evaluated. At first, the evaluation method used in the present invention will be explained.
Initially, the formulations of cosmetics (sunscreen and strongly-coating foundation) used in the tests will be shown.

### Formulation of sunscreen

| | |
|---|---|
| (1) Methyl polysiloxane | 5.0 |
| (2) Decamethylcyclopentasiloxane | 20.0 |
| (3) Trimethylsiloxysilicate | 2.0 |
| (4) Polyoxyethylene Methyl polysiloxane copolymer | 1.0 |
| (5) 1,3-butylene glycol | 5.0 |
| (6) Isostearic acid | 0.3 |
| (7) Titanium oxide | 17.0 |
| (8) Octyl methoxycinnamate | 8.0 |
| (9) Clay mineral | 0.5 |
| (10) Polyalkyl acrylate | 5.0 |
| (11) Trisodium edetate | proper quantity |
| (12) Antiseptic | proper quantity |
| (13) Perfume | proper quantity |
| (14) Purified water | reminder |

### Formulation of foundation

| | |
|---|---|
| (1) Decamethylcyclopentasiloxane | 14.0 |
| (2) Octamthylcyclotetrasiloxane | 24.0 |
| (3) Siliconated pullulan | 15.0 |
| (4) Isostearic acid | 1.0 |
| (5) Titanium oxide | 5.0 |
| (6) Octyl methoxycinnamate | 5.0 |
| (7) Dextrin fatty acid coated powder | 25.0 |
| (8) Alcohol | reminder |
| (9) Purified water | proper quantity |

For evaluations (1) to (5) listed below, the below-described composition was prepared, as the control skin cleanser, and used as the evaluation standard. Composition of the control skin cleanser

| | |
|---|---|
| (1) Carboxyvinylpolymer | 0.5 mass % |
| (2) Hydroxyethylcellulose | 0.02 |
| (3) Acrylic acid / alkyl Methacrylate alkyl copolymer | 0.1 |
| (4) Decamethylcyclopentasiloxane | 10.0 |
| (5) Liquid paraffin | 3.0 |
| (6) POE(10) isostearic acid | 5.0 |
| (7) 1,3-butylene glycol | 5.0 |
| (8) Potassium hydroxide | proper quantity |
| (9) Perfume | proper quantity |
| (10) Purified water | reminder |

### (Preparation method)

Components (1) to (3) and components (6) to (8) were uniformly mixed in component (10) and dissolved, and an oil phase component obtained by mixing components (4) and (5) was mixed into the solution with stirring. Component (9) was further added to the mixture and emulsified with a homomixer, and a creamy cleansing agent (O/W type) was obtained.

### "Evaluation (1): Mixability of a skin cleanser and cosmetics"

Both a sunscreen and a strongly-coating foundation were applied, the face was cleansed with a test sample after 2 hours, and the actual usage test by 10 professional panelists, for the mixability of the skin cleanser and the cosmetics, was conducted. The score based on the below-described rating criteria was determined. Here, the score was determined by setting the score of the mixability of the control skin cleanser and the cosmetics to be zero. The average value was calculated by dividing the sum of scores of each panelist by the number of panelists, and the evaluation results were obtained based on the below-described evaluation criteria.

### Rating criteria

+3: Very good mixability was recognized compared with the control skin cleanser.
+2: Good mixability was recognized compared with the control skin cleanser.
+1: Some mixability was recognized compared with the control skin cleanser.
0: Neither applies.
-1: The mixability was hardly present compared with the control skin cleanser.
-2: No mixability was present compared with the control skin cleanser.
-3: Absolutely no mixability was present compared with the control skin cleanser.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### "Evaluation (2): Rinsability of the skin cleanser"

Both a sunscreen and a strongly-coating foundation were applied, the face was cleansed with a test sample after 2 hours, and the actual usage test by 10 professional panelists, for the rinsability of the skin cleanser, was conducted. The score based on the below-described rating criteria was determined. Here, the score was determined by setting the score of the rinsability of the control skin cleanser to be zero. The average value was calculated by dividing the sum of scores of each panelist by the number of panelists, and the evaluation results were obtained based on the below-described evaluation criteria.

### Rating criteria

+3: Very good rinsability was recognized compared with the control skin cleanser.
+2: Good rinsability was recognized compared with the control skin cleanser.
+1: Some rinsability was recognized compared with the control skin cleanser.
0: Neither applies.
-1: The rinsability was hardly present compared with the control skin cleanser.
-2: No rinsability was present compared with the control skin cleanser.
-3: Absolutely no rinsability was present compared with the control skin cleanser.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### "Evaluation (3): Absence of sticky feeling after cleansing"

Both a sunscreen and a strongly-coating foundation were applied, the face was cleansed with a test sample after 2 hours, and the actual usage test by 10 professional panelists, for the sticky feeling after cleansing, was conducted. The score based on the below-described rating criteria was determined. Here, the score was determined by setting the score of the sticky feeling after the use of the control skin cleanser to be zero. The average value was calculated by dividing the sum of scores of each panelist by the number of panelists, and the evaluation results were obtained based on the below-described evaluation criteria.

### Rating criteria

+3: Absolutely no sticky feeling was recognized after cleansing compared with the control skin cleanser.
+2: No sticky feeling was recognized after cleansing compared with the control skin cleanser.
+1: Almost no sticky feeling was recognized after cleansing compared with the control skin cleanser.
0: Neither applies.
-1: Some sticky feeling was present after cleansing compared with the control skin cleanser.
-2: Sticky feeling was present after cleansing compared with the control skin cleanser.
-3: A significant sticky feeling was present after cleansing compared with the control skin cleanser.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### "Evaluation (4): Frictional feeling after cleansing"

Both a sunscreen and a strongly-coating foundation were applied, the face was cleansed with a test sample after 2 hours, and the actual usage test by 10 professional panelists, for the frictional feeling after cleansing, was conducted. The score based on the below-described rating criteria was determined. Here, the score was determined by setting the score of the frictional feeling of the control skin cleanser to be zero. The average value was calculated by dividing the sum of scores of each panelist by the number of panelists, and the evaluation results were obtained based on the below-described evaluation criteria.

### Rating criteria

+3: Absolutely no frictional feeling was recognized after cleansing compared with the control skin cleanser.
+2: No frictional feeling was recognized after cleansing compared was recognized with the control skin cleanser.
+1: Almost no frictional feeling was recognized after cleansing compared with the control skin cleanser.
0: Neither applies.
-1: Some frictional feeling was present after cleansing compared with the control skin cleanser.
-2: A frictional feeling was present after cleansing compared with the control skin cleanser.
-3: A significant frictional feeling was present after cleansing compared with the control skin cleanser.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### "Evaluation (5): Cosmetic removing effect"

Both a sunscreen and a strongly-coating foundation were applied, the face was cleansed with a test sample after 2 hours, and the actual usage test by 10 professional panelists, for the cosmetic removing effect after cleansing, was conducted. The score based on the below-described rating criteria was determined. Here, the score was determined by setting the score of the removing effect by the control skin cleanser to be zero. The average value was calculated by dividing the sum of scores of each panelist by the number of panelists, and the evaluation results were obtained based on the below-described evaluation criteria.

### Rating criteria

+3: A very high cosmetic removing effect was recognized after cleansing compared with the control skin cleanser.
+2: A high cosmetic removing effect was recognized after cleansing compared with the control skin cleanser.
+1: Somewhat high cosmetic removing effect was recognized after cleansing compared with the control skin cleanser.
0: Neither applies.
-1: Somewhat low cosmetic removing effect was recognized after cleansing compared with the control skin cleanser.
-2: A low cosmetic removing effect was recognized after cleansing compared with the control skin cleanser.
-3: A very low cosmetic removing effect was recognized after cleansing compared with the control skin cleanser.

### Evaluation criteria

A: The average value of 10 panelists was +1.5 points or higher.
B: The average value of 10 panelists was 0 point or higher and less than 1.5 points.
C: The average value of 10 panelists was -1.5 points or higher and less than 0 point.
D: The average value of 10 panelists was less than -1.5 points.

### "Evaluation (6): Skin Irritation Test"

A 24-hour occlusive patch test was performed on the medial side of the upper arm of 10 subjects, and then the average value was calculated based on the following rating criteria. The evaluation criteria were as described below.

### Rating criteria

0 point: No abnormality was observed.
1 point: Slight redness was observed.
2 points: Redness was observed.
3 points: Redness and papules were observed.

### Evaluation criteria

A: The average value of 10 panelists was less than 0.15 points.
B: The average value of 10 panelists was 0.15 points or higher and less than 0.2 points.
C: The average value of 10 panelists was 0.2 points or higher and less than 0.3 points.
D: The average value of 10 panelists was 0.3 points or higher.

The present inventors have actually prepared skin cleansers using the below-described test-use basic composition containingvarious alkylene oxide derivatives and conducted the evaluation thereof. The face was cleansed with the below-described cleansing agent of the basic composition by directly applying the cleansing agent on the face and then rinsing with water.

### Basic composition for test

| | |
|---|---|
| (1) Carboxyvinyl polymer | 0.5 mass % |
| (2) Hydroxyethyl cellulose | 0.02 |
| (3) Acrylic acid-alkyl methacrylate copolymer | 0.1 |
| (4) Decamethylcyclopentasiloxane | 10.0 |
| (5) Liquid paraffin | 3.0 |
| (6) Alkylene oxide derivative | |
| (7) Moisturizer | |
| (8) Potassium hydroxide | proper quantity |
| (9) Perfume | proper quantity |
| (10) Purified water | reminder |

### (Preparation method)

Components (1) to (3) and components (6) to (8) were uniformly mixed in component (10) and dissolved, and an oil phase component obtained by mixing components (4) and (5) was mixed into the solution with stirring. Component (9) was further added to the mixture and emulsified with a homomixer, and a creamy cleansing agent (O/W type) was obtained.

The present inventors have investigated various skin cleansers containingvarious alkylene oxide derivatives. The results are shown in Tables 4 to 6.
A block-type alkylene oxide derivative in the below-described table has a structure of the below-described formula (II). In the case that a+c+e=30 and b+d+f=30, for example, it will be denoted by (BO)₃₀(EO)₃₀.

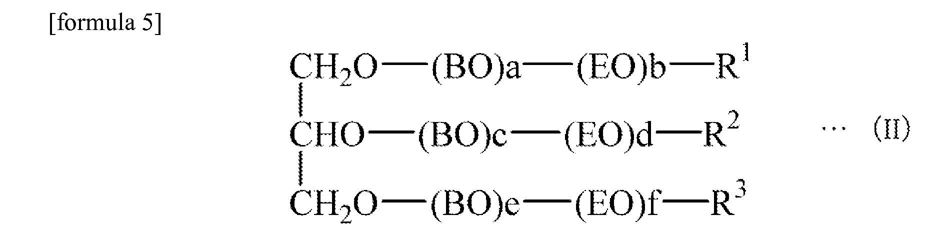

**[Table 4]**

| | Test Example | | | | | | | | | | |
|---|---|---|---|---|---|---|---|---|---|---|---|
| | control | 1 | 2 | 3 | 4 | 5 | 6 | 7 | 8 | 9 | 10 |
| POE(10) isostearic acid | 5.0 | - | - | - | - | - | - | - | - | - | - |
| (BD)₁₄(EO)₃₄, R^{1∼3}=CH₃, block polymer | - | 5.0 | - | - | - | - | - | - | - | - | - |
| (BO)₂₂(EO)₅₅, R^{1∼3}=CH₃, block polymer | - | - | 5.0 | - | - | - | - | - | - | - | - |
| (BO)₃₀(EO)₃₀ R^{1∼3}=CH₃, block polymer | - | - | - | 5.0 | - | - | - | - | - | - | - |
| (BO)₄₀(EO)₈₀, R^{1∼3}=CH₃, block polymer | - | - | - | - | 5.0 | - | - | - | - | - | - |
| (BO)₈₀(EO)₄₀, R^{1∼3}=CH₃, block polymer | - | - | - | - | - | 5.0 | - | - | - | - | - |
| (BO)₃₀(EO)₃₀, R^{1∼3}=C₄H₉, block polymer | - | - | - | - | - | - | 5.0 | - | - | - | - |
| (EO)₅₇, R^{1∼3}=CH₃ | - | - | - | - | - | - | - | 5.0 | - | - | - |
| (BO)₃₄, R^{1∼3}=CH₃ | - | - | - | - | - | - | - | - | 5.0 | - | - |
| (BO)₃₀(EO)₃₀, R^{1∼3}=H, block polymer | - | - | - | - | - | - | - | - | - | 5.0 | - |
| (BO)₃₀(EO)₃₀, R^{1∼3}=H, random polymer | - | - | - | - | - | - | - | - | - | - | 5.0 |
| Evaluation (1): Mixability of a skin cleanser and cosmetics | A | A | A | A | B | A | A | C | C | A | B |
| Evaluation (2): Rinsability of the skin cleanser | A | A | A | A | B | A | A | D | D | A | A |
| Evaluation (3): Absence of sticky feeling after cleansing | C | A | A | A | A | B | B | B | D | C | B |
| Evaluation (4): Frictional feeling after cleansing | C | A | A | A | A | B | B | C | B | C | A |
| Evaluation (5): Cosmetic removing effect | C | A | A | A | A | B | B | C | C | A | C |
| Evaluation (6): Skin Irritation Test | C | A | A | A | A | B | B | B | C | B | A |

In Test Examples 1 to 10 in Table 4, as a moisturizer, 5 mass % of 1,3-butylene glycol was blended.
As the control, when the traditionally used surfactant POE (10) isostearate was blended for cleansing property (control), the sticky feeling and skin irritation after cleansing were not satisfactory.
When the blended alkylene oxide derivative consists of only the oxyethylene part or only the oxybutylene part (Test Examples 7 and 8), the mixability with cosmetics, rinsability during cleansing, and the cosmetic removing effect were very poor because these materials do not function as a surfactant.

In Test Example 9 in which an alkylene oxide derivative with terminal hydrogen atoms is blended, the evaluation in after-cleansing sticky feeling and skin irritation was poor. When a random-type alkylene oxide derivative was blended (Test Example 10), the mixability with cosmetics, rinsability during cleansing, and the cosmetic removing effect were not satisfactory because the function as a surfactant is low.
On the other hand, the test examples in which various block-type alkylene oxide derivatives were blended (Test Examples 1 to 6) were excellent in all evaluations (1) to (6).

As is clear from the above-described results, when a block-type alkylene oxide derivative with a specific structure is blended in a skin cleanser, a skin cleanser having good mixability with cosmetics, excellent in usability such as easy rinsing during cleansing and no sticky feeling and frictional feeling after cleansing, excellent in the cosmetic removing effect, and low in skin irritation, can be obtained.
In the following, the moisturizerblended in the skin cleanser was investigated. The results are shown in Table 5.

**[Table 5]**

| | Test Example | | | | |
|---|---|---|---|---|---|
| | 11 | 12 | 13 | 14 | 15 |
| (BO)₁₄(ED)₃₄, R^{1∼3}=CH₃, block polymer | 5.0 | 5.0 | 5.0 | - | 5.0 |
| Propylene glycol | 5.0 | - | - | 5.0 | - |
| Glycerin | - | 5.0 | - | - | - |
| 1,3-butylene glycol | - | - | 5.0 | - | - |
| Evaluation (1): Mixability of a skin cleanser and cosmetics | A | A | A | D | B |
| Evaluation (2): Rinsability of the skin cleanser | A | A | A | C | B |
| Evaluation (3): Absence of sticky feeling after cleansing | A | A | A | B | A |
| Evaluation (4): Frictional feeling after cleansing | A | A | A | C | C |
| Evaluation (5): Cosmetic removing effect | A | A | A | D | B |
| Evaluation (6): Skin Irritation Test | A | A | A | C | A |

When a block-type alkylene oxide derivative with a specific structure was not blended (Test Example 14), it was recognized to be poor in the rinsability during cleansing, frictional feeling after cleansing, and skin irritation. When a moisturizer such as propylene glycol or glycerin was not blended (Test Example 15), the frictional feeling after cleansing was recognized to be especially poor.
On the other hand, when a combination of a block-type alkylene oxide derivative with a specific structure and a moisturizer was blended (Test Examples 11 to 13), they were excellent in all evaluations (1) to (6).
In the following, the preferable blending quantity, in the skin cleanser, of a block-type alkylene oxide derivative with a specific structure was investigated, and the results are shown in Table 6

**[Table 6]**

| | Test Example | | | |
|---|---|---|---|---|
| | 16 | 17 | 18 | 19 |
| (BO)₁₄(EO)₃₄ R^{1∼3}=CH₃, block polymer | 0.1 | 1.0 | 10.0 | 20 |
| 1,3-butylene glycol | 5.0 | 5.0 | 5.0 | 5.0 |
| Evaluation (1): Mixability of a skin cleanser and cosmetics | B | A | A | B |
| Evaluation (2): Rinsability of the skin cleanser | B | A | A | B |
| Evaluation (3): Absence of sticky feeling after cleansing | B | A | A | B |
| Evaluation (4): Frictional feeling after cleansing | B | A | A | A |
| Evaluation (5): Cosmetic removing effect | B | A | A | A |
| Evaluation (6): Skin Irritation Test | B | A | A | A |

From the results of Table 6, the effects could be observed from a blending quantity of a block-type alkylene oxide derivative with a specific structure of about 0.1 mass %, and especially significant effects could be observed at 1.0 mass % or higher. If the blending quantity is 20.0 mass % or higher, some sticky feeling after cleansing etc. starts to be generated; thus the blending up to about 10 mass % is especially preferable. Accordingly, the preferable blending quantity of a block-type alkylene oxide derivative with a specific structure is 0.1 to 20.0 mass %.

In the following, some synthesis examples of various alkylene oxide derivatives blended in the skin cleansers of the above-described test examples are shown.

### <Synthesis Example 1>

### Synthesis of polyoxybutylene(30 mol)polyoxyethylene(30 mol)trimethylglyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.
The hydroxyl value of the compound that was obtained by purifying a sample taken before the methyl chloride reaction was 49, and the hydroxyl value of alkylene oxide derivative 1 was 0.4. The ratio of the number of terminal hydrogen atoms and the number of the terminal methyl groups was 0.008; thus the hydrogen atoms are almost completely replaced with methyl groups.

### <Synthesis Example 2>

### Synthesis of polyoxyethylene(57 mol)trimethylglyceryl ether (alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2508 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water.In addition, filtration was conducted to remove the salt formed after the treatment, and the alkylene oxide derivative was obtained.
The hydroxyl value of the compound that was obtained by purifying a sample taken before the methyl chloride reaction was 66, and the hydroxyl value of alkylene oxide derivative 1 was. The ratio of the number of terminal hydrogen atoms and the number of the terminal methyl groups was 0.60.009; thus the hydrogen atoms are almost completely replaced with methyl groups.

### <Synthesis Example 3>

### Synthesis of polyoxybutylene(30 mol)polyoxyethylene(30 mol)glyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.

### <Synthesis Example 4>

### Synthesis of polyoxybutylene(30 mol)polyoxyethylene(30 mol)tributylglyceryl ether (block-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, 2160 g of butylene oxide was dropwise added from a dropping apparatus, and the mixture was stirred for 2 hours. Subsequently, 1320 g of ethylene oxide was dropwise added from a dropping apparatus, and the mixture was stirred for 2 hours. Then, 800 g of potassium hydroxide was loaded, the inside of the system was replaced with dry nitrogen, 1200 g of butyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the block-type alkylene oxide derivative was obtained.
The hydroxyl value of the compound that was obtained by purifying a sample taken before the methyl chloride reaction was 50, and the hydroxyl value of alkylene oxide derivative 1 was 1.5. The ratio of the number of terminal hydrogen atoms and the number of the terminal butyl groups was 0.03; thus the hydrogen atoms are almost completely replaced with butyl groups.

### <Synthesis Example 5>

### Synthesis of polyoxybutylene(30 mol)polyoxyethylene(30 mol)glyceryl ether (random-type alkylene oxide derivative)

Into an autoclave, 92g of glycerin and 18g of potassium hydroxide, which was used as a catalyst, were loaded. The air in the autoclave was replaced with dry nitrogen, and the catalyst was completely dissolved with stirring at 140 °C. Then, a mixture of 2160 g of butylene oxide and 1320 g of ethylene oxide was dropwise added from a dropping apparatus, and the mixture was stirred for 2 hours. Then, 400 g of potassium hydroxide was loaded, the inside of the system was replaced with dry nitrogen, 300 g of methyl chloride was pressured in at 80 to 130 °C, and the reaction was carried out for 5 hours. Subsequently, the reaction product was taken out from the autoclave, neutralized with hydrochloric acid to pH 6 to 7, and treated at 100 °C for 1 hour under a reduced pressure of -0.088 MPa (gauge pressure) in order to remove contained water. In addition, filtration was conducted to remove the salt formed after the treatment, and the random-type alkylene oxide derivative was obtained.
The hydroxyl value of the compound that was obtained by purifying a sample taken before the methyl chloride reaction was 47, and the hydroxyl value of the obtained compound, random-type alkylene oxide derivative 5, was 0.5. The ratio of the number of terminal hydrogen atoms and the number of the terminal methyl groups was 0.011; thus the hydrogen atoms are almost completely replaced with methyl groups.

In the following, formulation examples of the skin cleanser of the present invention are listed; however, the technical scope of the present invention is not limited by these. The obtained skin cleanser was confirmed to have good mixability with cosmetics and confirmed to be excellent in usability such as easy rinsing during cleansing and no sticky feeling and frictional feeling after cleansing, excellent in the cosmetic removing effect, and low in skin irritation.

Blending examples are shown in the following.

### Blending Example 1: Makeup cleansing gel

| | |
|---|---|
| (1) Hydroxyethylcellulose | 0.1 mass % |
| (2) Carboxyvinylpolymer | 0.4 |
| (3) Acrylic acid / alkyl Methacrylate alkyl copolymer | 0.2 |
| (4) Trisodium edetate | proper quantity |
| (5) Sodium methyl cocoyl taurate | 0.1 |
| (6) Monoisosutearicacid polyethyleneglycol | 0.5 |
| (7) POB(30)POE(35) trimethyl glycerylether (block) | 5.0 |
| 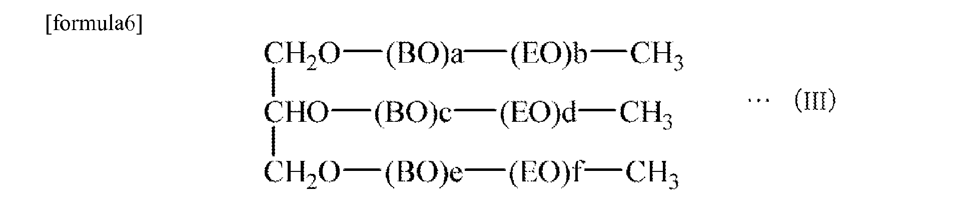 | |
| (In the formula (III), a+c+e =30, b+d+f=35, BO is an oxybuthylene group, EO is an oxyethylene group.) | |
| (8) Potassium hydroxide | proper quantity |
| (9) Alcohol | 5.0 |
| (10) Antiseptic | proper quantity |
| (11) Decamethylcyclopentasiloxane | 18.0 |
| (12) Methylpolysiloxane | 3.0 |
| (13) Perfume | proper quantity |
| (14) Purified water | reminder |

### (Process and evaluation)

Components (1) to (8) were added to component (14) and dissolved by stirring to obtain the water phase. Then, the solution in which component (10) was dissolved in component (9) was added to the water phase. Components (11) to (13) were further added, and the emulsification was carried out with an emulsifying machine to obtain a makeup cleansing gel. When the obtained makeup cleansing gel was directly applied to cosmetics and rinsed with water, the cleansing gel was recognized to have good mixability with cosmetics and confirmed to be excellent in usability such as easy rinsing during cleansing and no sticky feeling and frictional feeling after cleansing, excellent in the cosmetic removing effect, and low in skin irritation.

### Blending Example 2: Makeup cleansing gel

| | |
|---|---|
| (1) Hydroxyethylcellulose | 0.05 mass % |
| (2) Carboxyvinylpolymer | 0.45 |
| (3) Acrylic acid / alkyl Methacrylate alkyl copolymer | 0.1 |
| (4) Trisodium edetate | proper quantity |
| (5) Sodium methyl cocoyl taurate | 0.01 |
| (6) POB(32)POE(52) trimethyl glycerylether (block) | 7.0 |
| (In the formula (III), a+c+e =32, b+d+f=52, BO is an oxybuthylene group, EO is an oxyethylene group.) | |
| (7) Sodium polyaspartate solution | proper quantity |
| (8) Chamomilla extract | proper quantity |
| (9) Potassium hydroxide | proper quantity |
| (9) Alcohol | 5.0 |
| (10) Antiseptic | proper quantity |
| (11) Decamethylcyclopentasiloxane | 18.0 |
| (12) Methylpolysiloxane | 3.0 |
| (13) Perfume | proper quantity |
| (14) Purified water | reminder |

### (Process and evaluation)

Components (1) to (9) were added to component (16) and dissolved by stirring to obtain the water phase. Then, the solution in which component (11) to (12) was dissolved in component (10) was added to the water phase. Components (13) to (15) were further added, and the emulsification was carried out with an emulsifying machine to obtain a makeup cleansing gel. When the obtained makeup cleansing gel was directly applied to cosmetics and rinsed with water, the cleansing gel was recognized to have good mixability with cosmetics and confirmed to be excellent in usability such as easy rinsing during cleansing and no sticky feeling and frictional feeling after cleansing, excellent in the cosmetic removing effect, and low in skin irritation.

### Blending Example 3: Body shampoo

| | |
|---|---|
| (1) Hydroxypropylmethylcellulose | 0.1 mass % |
| (2) Glycerin | 10.0 |
| (3) Dipropyleneglycol | 5.0 |
| (4) Triethanolamine laurate | 12.0 |
| (5) Lauryl dimethylaminoaceticacid betaine | 5.0 |
| (6) Coconut fatty acid diethanolamide | 3.0 |
| (7) POB(17)POE(28) trimethyl glycerylether (block) | 5.0 |
| (In the formula (III), a+c+e =17, b+d+f=28, BO is an oxybuthylene group, EO is an oxyethylene group.) | |
| (8) Chamomilla extract | proper quantity |
| (9) Trisodium edetate | proper quantity |
| (10) Antiseptic | proper quantity |
| (11) Coloring material | proper quantity |
| (12) Perfume | proper quantity |
| (13) Purified water | reminder |

### (Process and evaluation)

Component (1) was added to component (13) and dispersed with stirring. The dispersion was heated to 70 °C, and components (2) to (12) were added and dissolved with stirring. Then, a body shampoo was obtained by cooling with a heat exchanger. When the obtained body shampoo was foamed with water, applied, and rinsed with water, the body shampoo was recognized to have good mixability with cosmetics and confirmed to be excellent in usability such as easy rinsing during cleansing and no sticky feeling and frictional feeling after cleansing, excellent in the cosmetic removing effect, and low in skin irritation.

### Blending Example 4: Milled soap

| | |
|---|---|
| (1) Sodium soap | reminder |
| (2) Potassium soap | 5.0 mass % |
| (3) Sodium chloride | 0.3 |
| (4) Glycerin | 0.5 |
| (5) Lauric acid | 5.0 |
| (6) POB(41)POE(48) trimethyl glycerylether (block) | 3.0 |
| (In the formula (III), a+c+e =41, b+d+f=48, BO is an oxybuthylene group, EO is an oxyethylene group.) | |
| (7) Coloring material | proper quantity |
| (8) Trisodium edetate | proper quantity |
| (9) Perfume | proper quantity |

### (Process and evaluation)

Components (1) to (9) were mixed with heating at 60 °C, and the solution was poured into a mold, cooled, and solidified to obtain milled soap. When the obtained soap was foamed with water, applied, and rinsed with water, the soap was recognized to have good mixability with cosmetics and confirmed to be excellent in usability such as easy rinsing during cleansing and no sticky feeling and frictional feeling after cleansing, excellent in the cosmetic removing effect, and low in skin irritation.

### Blending Example 5: Makeup cleansing gel

| | |
|---|---|
| (1) Hydroxyethylcellulose | 0.1 mass % |
| (2) Carboxyvinylpolymer | 0.3 |
| (3) Acrylic acid / alkyl Methacrylate alkyl copolymer | 0.3 |
| (4) Trisodium edetate | proper quantity |
| (5) Polyethylene glycol monoisosterate | 0.5 |
| (6) POB(14)POE(34) trimethyl glycerylether (block) | 3.0 |
| (In the formula (III), a+c+e =14, b+d+f=34, BO is an oxybuthylene group, EO is an oxyethylene group.) | |
| (7) Potassium hydroxide | proper quantity |
| (8) Alcohol | 5.0 |
| (9) POE hydrogenated castor oil | 0.3 |
| (10) Antiseptic | proper quantity |
| (11) Decamethylcyclopentasiloxane | 10.0 |
| (12) Methylpolysiloxane | 10.0 |
| (13) Perfume | proper quantity |
| (14) Purified water | reminder |

### (Process and evaluation)

Components (1) to (7) were added to component (14) and dissolved by stirring to obtain the water phase. Then, the solution in which component (9) to (10) was dissolved in component (8) was added to the water phase. Components (11) to (13) were further added, and the emulsification was carried out with an emulsifying machine to obtain a makeup cleansing gel. When the obtained makeup cleansing gel was directly applied to cosmetics and rinsed with water, the cleansing gel was recognized to have good mixability with cosmetics and confirmed to be excellent in usability such as easy rinsing during cleansing and no sticky feeling and frictional feeling after cleansing, excellent in the cosmetic removing effect, and low in skin irritation.

## Claims

1. A skin external preparation comprising a block-type alkylene oxide derivative represented by the below-described general formula (I):
(formula I) Y-[O(EO)ₐ-(AO)ₕ-(EO)_{c}-R]ₖ (I)
(In the formula, Y is the residue given by removing hydroxyl groups from a polyhydric alcohol having 3 to 6 hydroxyl groups, and k is the number of hydroxyl groups of the polyhydric alcohol. EO is an oxyethylene group, AO is an oxyalkylene group having 3 to 6 carbon atoms, and they are attached in a block-type, respectively. The products, a×k, b×k, and c×k, are the average addition mole numbers of the oxyethylene group, the oxyalkylene group having 3 to 6 carbon atoms, and the oxyethylene group, respectively, and 0≦a×k≦ 100, 1≦b×k≦100, and 0≦c×k≦100, however, (a+c)×k>1. The percentage of all oxyethylene groups in formula (I) with respect to the sum of all oxyethylene groups and oxyalkylene groups having 3 to 6 carbon atoms in formula (I) is 10 to 80 mass %. Rs may be either identical to or different from each other, and they are hydrocarbon groups with 1 to 4 carbon atoms.)

2. A skin external preparation according to claim 1, wherein AO of a block-type alkylene oxide derivative represented by the above-described formula (I) is an oxybutylene group.

3. A skin external preparation according to claim 1 or 2, wherein the number a of the block-type alkylene oxide derivative represented by the above-described formula (I) is zero and that the addition order of AO and EO is (AO)-(EO) with respect to Y in the formula.

4. A skin external preparation according to claim 1 to 3, comprising 0.01 to 70 mass % of a block-type alkylene oxide derivative represented by the above-described general formula (I).

5. A rough skin improving agent comprising a block-type alkylene oxide derivative represented by the above -described general formula (I).

6. A usability-improving agent comprising a block-type alkylene oxide derivative represented by the above -described general formula (I).

7. A skin cleanser comprising 0.01 to 70 mass % of a block-type alkylene oxide derivative represented by the above-described formula (I) and 0.1 to 20 mass % of a moisturizer.

8. A skin cleanser according to claim 7, wherein the content of a block-type alkylene oxide derivative with the above-described specific structure is 0.1 to 20 mass %.

9. A skin cleanser according to claim 7 or 8, wherein the AO group of a block-type alkylene oxide derivative represented by the above-described formula (I) is an oxybutylene group.

10. A skin cleanser according to claim 7 to 9, wherein the number a of the block-type alkylene oxide derivative represented by the above-described formula (I) is zero and that the addition order of AO and EO is (AO)-(EO) with respect to Y in the formula.

11. A skin cleanser according to claim 7 to 10, wherein the above-described moisturizer is one or more selected from the group consisting of dipropylene glycol, propylene glycol, 1,3-butylene glycol, and glycerin.
